# EUROPEAN PATENT SPECIFICATION

(11) **EP 1 450 775 B1**
(45) Date of publication and mention of the grant of the patent: **12.03.2008**
(21) Application number: 02775081.9
(22) Date of filing: 20.09.2002
(51) Int. Cl.: A61K 31/506, A61P 31/04

(54) **C-KIT INHIBITORS FOR TREATING BACTERIAL INFECTIONS**
C-KITHEMMER ZUR BEHANDLUNG VON BAKTERIELLEN INFEKTIONEN
INHIBITEURS DE C-KIT POUR TRAITER DES INFECTIONS BACTERIENNES

(30) Priority: 20.09.2001 US 323313 P
(43) Date of publication of application: 01.09.2004
(73) Proprietor: AB Science, 75008 Paris (FR)
(72) Inventor: MOUSSY, Alain, F-75006 Paris (FR); KINET, Jean-Pierre, Lexington, MA 02421 (US)
(74) Representative: Warcoin, Jacques
(86) International application number: PCT/IB2002/004251
(87) International publication number: WO 2003/035049

(56) References cited:
- WO-A-01/47507
- WO-A-98/18924
- WO-A1-99/51219
- GAMBACORTI-PASSERINI C ET AL: "ROLE OF ALPHA1 ACID GLYCOPROTEIN IN THE IN VIVO RESISTANCE OF HUMAN BCRABL+ LEUKEMIC CELLS TO THE ABL INHIBITOR ST1571" JOURNAL OF THE NATIONAL CANCER INSTITUTE, US DEPT. OF HEALTH, EDICATIONAND WELFARE, PUBLIC HEALTH, US, vol. 92, no. 20, 18 October 2000 (2000-10-18), pages 1641-1650, XP001036595 ISSN: 0027-8874
- HEINRICH MICHAEL C ET AL: "Inhibition of c-kit receptor tyrosine kinase activity by STI 571, a selective tyrosine kinase inhibitor" BLOOD, vol. 96, no. 3, 1 August 2000 (2000-08-01), pages 925-932, XP001097629 ISSN: 0006-4971
- MAURER M ET AL: "THE C-KIT LIGAND, STEM CELL FACTOR, CAN ENHANCE INNATE IMMUNITY THROUGH EFFECTS ON MAST CELLS" JOURNAL OF EXPERIMENTAL MEDICINE, TOKYO, JP, vol. 188, no. 12, 21 December 1998 (1998-12-21), pages 2343-2348, XP008014256 ISSN: 0022-1007
- KLIMPEL G R ET AL: "A ROLE FOR STEM CELL FACTOR (SCF): C-KIT INTERACTION(S) IN THE INTESTINAL TRACT RESPONSE TO SALMONELLA TYPHIMURIUM INFECTION" JOURNAL OF EXPERIMENTAL MEDICINE, TOKYO, JP, vol. 184, no. 1, 1 July 1996 (1996-07-01), pages 271-276, XP008014258 ISSN: 0022-1007
- SHIN J.-S.; ABRAHAM S.N.: 'Co-option of endocytic functions of cellular caveolae by pathogens' IMMUNOLOGY vol. 102, 2001, pages 2 - 7
- ABRAHAM S.N. ET AL: 'Type 1 Fimbriated E. coli-Mast Cell Interactions in Cystitis' JOURNAL OF INFECTIOUS DISEASES vol. 183, no. SUPPL.1, 2001, pages S51 - S55
- DATABASE BIOSIS [Online] BIOSCIENCES INFORMATION SERVICE, PHILADELPHIA, PA, US PREV200100299325, 16 November 2000 HEINRICH MICHAEL C. ET AL: 'STI 571 inhibits the kinase activity of wild type and juxtamembrane c-kit mutants but not the exon 17 D816V mutation'
- DATABASE BIOSIS [Online] BIOSCIENCES INFORMATION SERVICE, PHILADELPHIA, PA, US PREV200100311789, 16 November 2000 TATTON LOUISE; CHOPRA RAJ; KHWAJA ASIM: 'The selective src kinase inhibitor PP1 also inhibits c-kit and Bcr-Abl tyrosine kinases and induces apoptosis in mast cell leukemia, CML amd AML cells'

## Description

The present invention relates to the use of compounds according to formula II for treating recurrent bacterial infections, preferably infections caused by FimH expressing bacteria, whereby a tyrosine kinase inhibitor is administered to a human in need of such treatment, more particularly a non toxic, potent and selective c-kit inhibitor, wherein said inhibitor is unable to promote death of IL-3 dependent cells cultured in presence of IL-3 is administered.

Bacterial infections are the most common diseases among mammalian and yet they remain deadly in case of resistant strains appearance. Resistance primarily originates from the extensive use of antibiotics. Antibiotics are agents acting on the bacterial cell wall such as bacitracin, the cephalosporins, and the penicillins, agents capable of inhibiting replication and protein synthesis by their effects on ribosomes, such as the aminoglycosides, the tetracyclines, the streptomycins and the macrolide antibiotics such as erythromycin; agents affecting nucleic acid metabolism, such as the fluoroquinolones, actinomycin; and drugs affecting intermediary metabolism, such as the sulfonamides and trimethoprim.

Despite the efficacy of antibiotics, few bacteria occasionally acquire mutations under high selection pressure, which renders the above mentioned antibiotic molecular targets insensitive and leads to the birth of new resistant strains.
More recently, muti-resistant strains have been observed during nosocomial infections and has come to the attention of the public. Facing the emergence of these deadly strains, research has focused on other mechanisms leading to multi-resistance. For example, it has been found that the marA loci confers multiple antibiotic resistance via increased efflux of many structurally unrelated antibiotics (McMurry et al., Antimicrob. Agents Chemother. 38:542-546, 1994). Multi-drug efflux pumps are now generally thought to be responsible for drugs insensitivity.

However, this mechanism leading to the resistance of bacteria does not explain the recurrence observed in bacterial infections. Indeed, after eradication of the bacteria, resurgence is observed later on suggesting that a small portion of bacteria were able to survive and remain concealed in the body. For example, urinary tract infections (UTI) have been treated for years with the antibiotics Bactrim, Macrodantin and a combination of Sulfa drugs that offer quick relief, but these antibiotics become useless after several prescriptions because the infection looks as if it has settled in the body and re-emerges from time to time.

Therefore, there is a need for new medications that would prevent and treat resurgence of bacterial infections.

In connection with the invention, it is postulated that bacteria, especially FimH expressing bacteria, are capable of escaping the immune system as well as the action of antibiotics by integration into mast cells, in which they remain concealed for a period time.

Mast cells (MC) are tissue elements derived from a particular subset of hematopoietic stem cells that express CD34, c-kit and CD 13 antigens (Kirshenbaum et al, Blood. 94: 2333-2342, 1999 and Ishizaka et al, Curr Opin Immunol. 5: 937-43, 1993). Immature MC progenitors circulate in the bloodstream and differentiate in tissues. These differentiation and proliferation processes are under the influence of cytokines, one of utmost importance being Stem Cell Factor (SCF), also termed Kit ligand (KL), Steel factor (SL) or Mast Cell Growth Factor (MCGF). SCF receptor is encoded by the protooncogene c-kit, that belongs to type III receptor tyrosine kinase subfamily (Boissan and Arock, J Leukoc Biol. 67: 135-48, 2000). This receptor is also expressed on others hematopoietic or non hematopoietic cells. Ligation of c-kit receptor by SCF induces its dimerization followed by its transphosphorylation, leading to the recruitement and activation of various intracytoplasmic substrates. These activated substrates induce multiple intracellular signaling pathways responsible for cell proliferation and activation (Boissan and Arock, 2000). Mast cells are characterized by their heterogeneity, not only regarding tissue location and structure but also at the functional and histochemical levels (Aldenborg and Enerback., Histochem. J. 26: 587-96, 1994; Bradding et al. J lmmunol. 155: 297-307, 1995 ; Irani et al, J Immunol. 147: 247-53,1991 ; Miller et al, Curr Opin Immunol. 1: 637-42, 1989 and Welle et al, J Leukoc Biol. 61: 233-45, 1997).

Apart from their key role as effector cells of allergic and potentially lethal anaphylactic reactions, mast cells might contribute to the initiation of acquired immune reactions. Indeed, mast cells can phagocytosize diverse particles, and particularly bacteria. For example, recent studies have implicated rodent mast cells in the innate immune response to infectious bacteria and have shown that human mast cells are intrinsically capable of mediating microbial recognition and of actively contributing to the host defense against bacteria ; Arock M et al, Infect Immun 1998 Dec;66(12):6030-4. Galli SJ et al, Curr Opin Immunol 1999 Feb;11 (1):53-9 suggested that mast cell function can be manipulated for therapeutic ends using SCF to boost immune response. This was also proposed by Maurer et al, J Exp Med 1998 Dec 21;188(12):2343-8 who identified c-kit and mast cells as potential therapeutic targets for enhancing innate immune responses.

While this can be acknowledged as far as acute infections are concerned, it could have serious drawbacks when considering recurrent bacterial infections.

Indeed, mast cells display very peculiar cell membrane structures called caveolae. Caveolae are subcellular structures implicated in the import and transcytosis of macromolecules and in transmembrane signaling. The composition and function of caveolae is reviewed in Anderson RG, Annu Rev Biochem 1998;67:199-225. In this article, caveolae are presented not just as an endocytic device with a peculiar membrane shape but rather as an entire membrane system with multiple functions essential for the cell. It is also mentioned that pathogens have been identified that use it as a means of gaining entrance to the cell.

Shin JS et al, Science. 2000 Aug 4;289(5480):732-3 reported that caveolae were detected in the microvilli and intracellular vesicles of cultured mouse bone marrow-derived mast cells (BMMCs). CD48, a receptor for FimH-expressing (type 1 fimbriated) Escherichia coli, was specifically localized to plasmalemmal caveolae in BMMCs. The involvement of caveolae in bacterial entry into BMMCs was demonstrated because caveolae-disrupting and -usurping agents specifically blocked E. coli entry. More importantly, it was demonstrated that some microbes utilize the unique features of caveolae to enter and traffic, without any apparent loss of viability and function, to different sites within immune and other host cells; Shin & Abraham, Immunology 2001, 102 (1), 2-7.

Therefore, bacteria-encapsulating caveolar chambers in mast cells form a reservoir of surviving bacteria that is postulated here to be implicated in the resurgence of infections.

FimH, a mannose-binding lectin, expressed by many enterobacteria including *E. coli, K. pneumoniae* and *S. typhimurium,* binds to the receptor CD48 present at the surface of caveolae, Shin JS et al, FEMS Microbiol Lett 2001 Apr 13;197(2):131-8. As a result, FimH expressing bacteria enter inside mast cells and remain concealed and viable in caveolar chambers. In addition, Abraham SN et al, Nature 1988 Dec 15;336(6200):682-4 have observed a conservation of the D-mannose-adhesion protein among type 1 fimbriated members of the family Enterobacteriaceae.

It is proposed here that at some point, exocytosis of these chambers leads to the release of intact and living bacteria, which are responsible for the resurgence of the infection.

Consequently, apart from being beneficial for the organism through its ability to initiate immune responses towards a variety of pathogens (M. Maurer, et al., J. Exp. Med. Vol. 188(12), 1998, pages 2343-2348 and G.R. Klimpel, et al., J. Exp. Med. Vol. 184, 1996, pages 271-276), the mast cell may also be detrimental for the host during recurrent infectious diseases as specified above.

In such detrimental circumstances, therapeutic strategies aiming at blocking the activation and the survival of mast cells, for instance through inhibition of c-kit or c-kit signaling is proposed to decrease the inappropriate release of inflammatory mediators, as well as the survival of intracellular pathogens.

Compounds useful for treating resurging infection have been proposed in WO 99/51219. L. Tatton, et al., Blood, 2000, 42nd Annual Meeting of the American Society of Hematology, San Francisco, California, USA, December 01-05, 2000, Vol. 96, Nr. 11 part-1, Page 173b describes pyrazolopyrimidines as c-kit and Bcr-AbI inhibitors for treating myeloid malignancies. M. C. Heinrich, et al., Blood, Vol. 96(3), 2000, pages 925-932 and M.C. Heinrich, et al., Blood, 2000, 42nd Annual Meeting of the American Society of Hematology, San Francisco, California, USA, December 01-05, 2000, Vol. 96, Nr. 11 part 2, page 81 a describes the compound known as ST1571 as a PDGK/c-kit inhibitor useful for treating leukemia.

The invention provides a new therapeutic strategy aimed at the use of c-kit specific kinase inhibitors according to formula II to inhibit mast cell proliferation, survival and activation. A new route for treating recurrent bacterial infections is provided, which consists of destroying mast cells that constitute a reservoir for bacteria. It has been found among tyrosine kinase inhibitors, c-kit inhibitors according to formula II are especially suited to reach this goal.

### Description

The present invention relates to the use of a c-kit inhibitor selected from the group consisting of N-phenyl-2-pyrimidine-amine derivatives having the formula II below, for preparing a medicament for treating recurrent bacterial infections, more particularly resurging infections after an asymptomatic period, wherein bacteria are FimH expressing bacteria, such as Gram-negative enterobacteria such as *E. coli, Klebsiella pneumoniae, Serratia marcescens, Citrobactor freudii* and *Salmonella typhimurium*, wherein the c-kit inhibitor is administered sequentially or simultaneously with at least one antibiotic selected from bacitracin, the cephalosporins, the penicillins, the aminoglycosides, the tetracyclines, the streptomycins and the macrolide antibiotics such as erythromycin; the fluoroquinolones, actinomycin, the sulfonamides and trimethoprim.

The N-phenyl-2-pyrimidine-amine derivative is selected from the compounds corresponding to formula II : 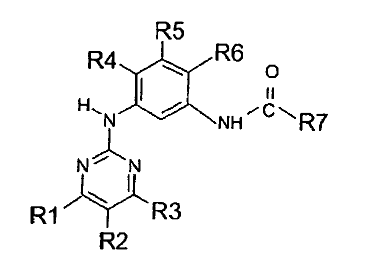
Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one basic site, such as an amino function.

Preferably, R7 is the following group :

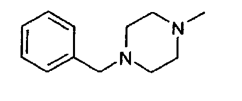

Among these compounds, the preferred are defined as follows:
R1 is a heterocyclic group, especially a pyridyl group,
R2 and R3 are H,
R4 is a C1-C3 alkyl, especially a methyl group,
R5 and R6 are H,
and R7 is a phenyl group bearing at least one substituent, which in turn possesses at least one basic site, such as an amino function, for example the group :

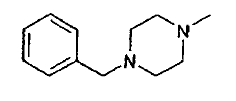

Therefore, in a preferred embodiment, the invention relates to the use of compounds according to formula II for treating recurrent bacterial infections comprising the administration of an effective amount of the compound known in the art as CGP57148B:
4-(4-mehylpiperazin-1-ylmethyl)-N-[4-methyl-3-(4-pyridine-3-yl)pyrimidine-2 ylamino)phényl]-benzamide corresponding to the following formula:

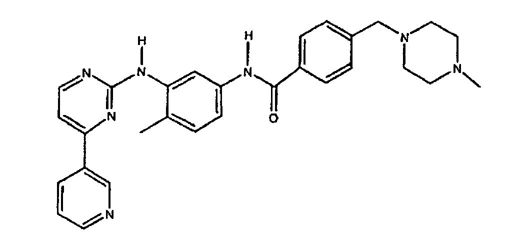

The preparation of this compound is described in example 21 of EP 564 409 and the β-form, which is particularly useful is described in WO 99/03854.

The expression "bacterial infections" will be understood herein as recurrent bacterial infections, more particularly resurging infections after asymptomatic periods. Preferably, bacteria are FimH expressing bacteria such as Gram-negative enterobacteria which include but are not limited to well known pathogenic species such as *E*. *coli, Klebsiella pneumoniae, Serratia marcescens, Citrobactor freudii* and *Salmonella typhimurium.* In connection with the invention, bacterial infections encompass recurrent urinary tract infections such as bacterial cystitis and respiratory tract infections.

Therefore, the invention embraces the use of the compounds defined above to manufacture a medicament for treating recurrent bacterial infections in mammalian, especially in human. Such medicament is particularly useful for the treatment of resurging infections after asymptomatic periods such as bacterial cystitis and respiratory tract infections. Preferably, the invention contemplates the use of the compounds defined above to manufacture a medicament for treating FimH expressing recurrent bacteria infections such as Gram-negative enterobacteria which include but are not limited to *E*. *coli, Klebsiella pneumoniae, Serratia marcescens, Citrobactor freudii* and *Salmonella typhimurium.*

The pharmaceutical compositions utilized in this invention may be administered by any number of routes including, but not limited to, oral, intravenous, intramuscular, intraarterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethylcellulose, or sodium carboxymethylcellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations which can be used orally include capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Pharmaceutical formulations suitable for parenteral administration may be formulated in aqueous solutions, preferably in physiologically compatible buffers such as Hanks' solution, Ringer's solution, or physiologically buffered saline. Aqueous injection suspensions may contain substances which increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers may also be used for delivery. Optionally, the suspension may also contain suitable stabilizers or agents which increase the solubility of the compounds to allow for the preparation of highly concentrated solutions.

The pharmaceutical composition may be provided as a salt and can be formed with many acids, including but not limited to, hydrochloric, sulfuric, acetic, lactic, tartaric, malic, and succine, acids, etc. Salts tend to be more soluble in aqueous or other protonic solvents than are the corresponding free base forms. In other cases, the preferred preparation may be a lyophilized powder which may contain any or all of the following: 1-50 mM histidine, 0. 1%-2% sucrose, and 2-7% mannitol, at a pH range of 4.5 to 5.5, that is combined with buffer prior to use.

Pharmaceutical compositions suitable for use in the invention include compositions wherein c-kit inhibitors according to formula II are contained in an effective amount to achieve the intended purpose. The determination of an effective dose is well within the capability of those skilled in the art. A therapeutically effective dose refers to that amount of active ingredient, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions which exhibit large therapeutic indices are preferred. As mentioned above, a c-kit inhibitor according to the invention is unable to promote death of IL-3 dependent cells cultured in presence of IL-3.

In another embodiment, the invention is aimed at the use of a combination comprising a c-kit inhibitor, and at least one antibiotic selected bacitracin, the cephalosporins, the penicillins, the aminoglycosides, the tetracyclines, the streptomycins and the macrolide antibiotics such as erythromycin; the fluoroquinolones, actinomycin, the sulfonamides and trimethoprim for sequential or simultaneous use for treating recurrent bacterial infections, resurging infections after asymptomatic periods such as bacterial cystitis and respiratory tract infections. This combination is particularly useful for treating FimH expressing bacteria infections such as Gram-negative enterobacteria including *E. coli, Klebsiella pneumoniae, Serratia marcescens, Citrobactor freudii* and *Salmonella typhimurium.* Preferably, said inhibitor is unable to promote death of IL-3 dependent cells cultured in presence of IL-3 and the combination further comprises an acceptable pharmaceutical carrier suitable for oral administration.

## Claims

1. The use of a c-kit inhibitor selected from the group consisting of N-phenyl-2-pyrimidine-amine derivatives having the formula II: 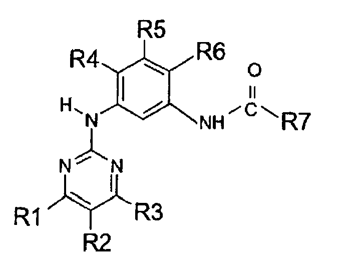
Wherein R1, R2 and R3 are independently chosen from H, F, CI, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one amino group;
for preparing a medicament for treating recurrent bacterial infections, more particularly resurging infections after an asymptomatic period, wherein bacteria are FimH expressing bacteria, such as Gram-negative enterobacteria such as *E*. *coli, Klebsiella pneumoniae, Serratia marcescens, Citrobactor freudii* and *Salmonella typhimurium*, wherein the c-kit inhibitor is administered sequentially or simultaneously with at least one antibiotic selected from bacitracin, the cephalosporins, the penicillins, the aminoglycosides, the tetracyclines, the streptomycins and the macrolide antibiotics such as erythromycin; the fluoroquinolones, actinomycin, the sulfonamides and trimethoprim.

2. The use according to claim 1, wherein R7 is

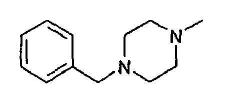

3. The use according to claim 2, wherein said inhibitor is the 4-(4-mehylpiperazine-1-ylmethyl)-N-[4-methyl-3-(4-pyridine-3-yl)pyrimidine-2 ylamino)phenyl]-benzamide.

4. The use according to one of claims 1 to 3 for treating urinary tract infections such as bacterial cystitis and respiratory tract infections.

5. The use according to one of claims 1 to 4 wherein said medicament is suitable for oral administration.

## Patentansprüche

1. Verwendung eines c-kit-Inhibitors, ausgewählt aus der Gruppe bestehend aus N-Phenyl-2-pyrimidinamin-Derivaten der Formel II: 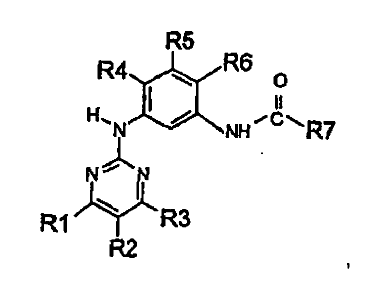
worin R1, R2 und R3 unabhängig aus H, F, Cl, Br, I, einer C1-C5-Alkylgruppe oder einer cyclischen oder heterocyclischen Gruppe, insbesondere einer Pyridylgruppe ausgewählt werden; R4, R5 und R6 unabhängig aus H, F, Cl, Br, I, einer C1-C5-Alkyl-, insbesondere einer Methylgruppe ausgewählt werden,
und R7 eine Phenylgruppe, welche wenigstens einen Substituenten, der seinerseits wenigstens eine Aminogruppe aufweist, trägt, ist;
zur Herstellung eines Arzneimittels zur Behandlung von wiederkehrenden bakteriellen Infektionen, insbesondere wieder auflebenden Infektionen nach einem symptomlosen Zeitraum, wobei die Bakterien FimH exprimierende Bakterien, wie Gram-negative Enterobakterien, wie *E*. *coli, Klebsiella pneumoniae, Serratia marcescens, Citrobacter freudii* und *Salmonella typhimurium,* sind, wobei der c-kit-Inhibitor nacheinander oder gleichzeitig verabreicht wird mit wenigstens einem Antibiotikum, welches aus Bacitracin, den Cephalosporinen, den Penicillinen, den Aminoglycosiden, den Tetracyclinen, den Streptomycinen und den Makrolid-Antibiotika, wie Erythromycin, den Fluorquinolonen bzw. Fluorchinolonen, Actinomycin, den Sulfonamiden und Trimethoprim ausgewählt wird.

2. Verwendung nach Anspruch 1, wobei R7 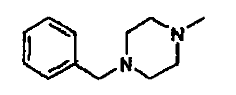 ist.

3. Verwendung nach Anspruch 2, wobei der Inhibitor 4-(4-Methylpiperazin-1-ylmethyl)-N-[4-methyl-3-(4-pyridin-3-yl)pyrimidin-2-ylamino)phenyl]-benzamid ist.

4. Verwendung nach einem der Ansprüche 1 bis 3 zur Behandlung von Harnwegsinfektionen, wie bakterieller Zystitis, und Atemwegsinfektionen.

5. Verwendung nach einem der Ansprüche 1 bis 4, wobei das Arzneimittel zur oralen Verabreichung geeignet ist.

## Revendications

1. Utilisation d'un inhibiteur de c-kit choisi dans le groupe constitué par les dérivés de N-phényl-2-pyrimidine-amine de formule II: 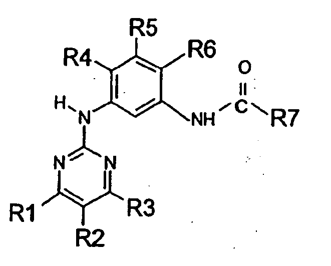
dans laquelle R1, R2 et R3 sont indépendamment choisis parmi H, F, C1, Br, I, un alkyle en C1-C5 ou un groupe cyclique ou hétérocyclique, en particulier un groupe pyridyle;
R4, R5 et R6 sont indépendamment choisis parmi H, F, Cl, Br, I, un alkyle en C1-C5, en particulier un groupe méthyle ;
et R7 est un groupe phényle portant au moins un substituant, qui possède à son tour au moins un groupe amino;
dans la préparation d'un médicament destiné à traiter les infections bactériennes récurrentes, plus particulièrement les infections résurgentes après une période asymptomatique, où les bactéries sont des bactéries exprimant FimH, comme les entérobactéries Gram négatives comme *E. coli, Klebsiella pneumoniae, Serratia marcescens, Citrobacter freundii* et *Salmonella typhimurium,* où l'inhibiteur de c-kit est administré séquentiellement ou simultanément avec au moins un antibiotique choisi parmi la bacitracine, les céphalosporines, les pénicillines, les aminoglycosides, les tétracyclines, les streptomycines et les antibiotiques macrolides comme l'érythromycine, les fluoroquinolones, l'actinomycine, les sulfamidés et le triméthoprime.

2. Utilisation selon la revendication 1, dans laquelle R7 est

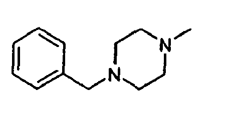

3. Utilisation selon la revendication 2, dans laquelle ledit inhibiteur est le 4-(4-méthylpipéraz ine-1-ylméthyl)-N-[4-méthyl-3-(4-pyridine-3-yl)pyrimidine-2-ylamino)phényl]-benzamide.

4. Utilisation selon l'une quelconque des revendications 1 à 3 pour traiter les infections du tractus urinaire comme la cystite bactérienne et les infections des voies respiratoires.

5. Utilisation selon l'une quelconque des revendications 1 à 4 dans laquelle ledit médicament est approprié pour être administré par voie orale.


### SEQUENCE LISTING

<110> AB Science
<120> Use of potent, selective and non toxic c-kit inhibitors
   for treating bacterial infections
<130> D19831 NT
<150> US 60/323,313
   <151> 2001-09-20
<160> 5
<170> Patent In Ver. 2.1
<210> 1
   <211> 976
   <212> PRT
   <213> Homo sapiens
<220>
   <223> Human c-kit
<400> 1
<210> 2
   <211> 30
   <212> DNA
   <213> Homo sapiens
<220>
   <223> Primer
<400> 2
   aagaagagat ggtacctcga ggggtgaccc 30
<210> 3
   <211> 33
   <212> DNA
   <213> Homo sapiens
<220>
   <223> Primer
<400> 3
   ctgcttcgcg gccgcgttaa ctcttctcaa cca 33
<210> 4
   <211> 20
   <212> DNA
   <213> Homo sapiens
<220>
   <223> Primer
<400> 4
   agctcgttta gtgaaccgtc 20
<210> 5
   <211> 20
   <212> DNA
   <213> Homo sapiens
<220>
   <223> Primer
<400> 5
   gtcagacaaa atgatgcaac 20